# EUROPÄISCHE PATENTSCHRIFT

(11) **EP 2 073 135 B1**
(45) Veröffentlichungstag und Bekanntmachung des Hinweises auf die Patenterteilung: **03.10.2018**
(21) Anmeldenummer: 07024884.4
(22) Anmeldetag: 21.12.2007
(51) Int. Cl.: G06F 19/00

(54) **Blutglukosesystem mit Zeitsynchronisation**
Blood glucose system with time synchronisation
Système de détermination du taux de glycémie à synchronisation temporelle

(43) Veröffentlichungstag der Anmeldung: 24.06.2009
(73) Patentinhaber: Roche Diabetes Care GmbH, 68305 Mannheim (DE); F. Hoffmann-La Roche AG, 4070 Basel (CH)
(72) Erfinder: Köhler, Matthias, 69514 Laudenbach (DE); Blasberg, Peter, 69469 Weinheim (DE)

(56) Entgegenhaltungen:
- EP-A- 1 758 039
- WO-A-2005/043306
- DE-A1- 19 733 445
- US-A- 5 921 938
- US-A1- 2005 103 351

## Beschreibung

Die Erfindung betrifft ein Blutglukosesystem zum Behandeln einer Glukosestoffwechselstörung mit einem Dosiergerät zum Abgeben eines medizinischen Wirkstoffs für die Behandlung der Glukosestoffwechselstörung an einen Körper, einem Blutglukosemessgerät zum Bestimmen des Blutglukosegehaltes des Körpers und einer Datenverarbeitungseinrichtung zum Verarbeiten von gespeicherten Datensätzen des Dosiergerätes und des Blutglukosemessgerätes. Die Erfindung richtet sich also auf Geräte für die Diagnose oder Behandlung einer Glukosestoffwechselstörung wie Diabetes.

Das Dosiergerät zum Abgeben eines medizinischen Wirkstoffs für die Behandlung der Glukosestoffwechselstörung an einen Körper ist vorzugsweise ein Injektionsgerät, vorzugsweise ein Gerät zum Abgeben oder Injizieren von Insulin. Solche Geräte sind beispielsweise als Insulinpen (beispielsweise von Novonordisc und J&J), als Insulinpumpe (beispielsweise von Roche) oder als Insulin-Inhalatoren bekannt. Bei Insulinpens handelt es sich um dicke Stifte mit einer Nadel und einer Insulinkartusche zum Abgeben von mehreren Einzeldosen an Insulin, wobei jedes Mal ein neuer Einstich erfolgt. Die bei einem Einstich abzugebende Anzahl an Einheiten von Insulin wird durch ein Einstellrad eingestellt. Aus der US 5,925,021 und der DE 695 34 225 T2 sind Insulinpen-Injektionsgeräte mit einem integrierten Zeitnormal bekannt.

Eine Insulinpumpe ist ein medizinisches Gerät zur kontinuierlichen oder intervallartigen subkutanen Insulininfusion. Es ist ein kleines Infusionsgerät, das am Körper getragen wird und das über einen Katheter und einer unter der Haut liegenden Nadel dem Körper kontinuierlich oder in Intervallen Insulin zuführt. Dabei kann die Dosierung auch einem bestimmten Tagesrhythmus oder bestimmten Ereignissen, wie beispielsweise der Einnahme von Mahlzeiten, angepasst werden.

Ein Blutglukosemessgerät im Rahmen der Erfindung ist ein analytisches Messgerät zum Bestimmen oder Aufzeichnen (so genannte "elektronische Tagebücher") eines Blutglukosewertes. Solche Geräte werden auch als Blutglukosemeter, Blutzuckermessgerät oder Blutglukoserecorder bezeichnet. Blutglukoserecorder sind Geräte, die über eine vorgegebene Zeitdauer Blutglukosekonzentrationen aufzeichnen, beispielsweise um für einen Diabetes-Patienten ein geeignetes Insulindosierungsschema festlegen zu können.

Ein Blutglukosemessgerät ist ein Gerät, mit dessen Hilfe der Blutzuckergehalt bestimmt werden kann. In der Regel wird hierzu in einem Körper eine Einstichwunde erzeugt, ein Bluttropfen entnommen und mit Hilfe des Blutzuckermessgerätes der Blutglukosegehalt in dem Tropfen bestimmt. Es ist aber auch denkbar, durch eine permanente Messung, beispielsweise mit in den Körper eingeführten Sensoren oder durch die Haut hindurch die Blutglukose zu messen.

Besonders bevorzugt werden im Rahmen der Erfindung Dosiergeräte und Blutglukosemessgeräte benutzt, die eigenständig, d.h. unabhängig von anderen angeschlossenen Komponenten oder Geräten funktionsfähig und betreibbar sind. Dies bedeutet beispielsweise, dass ein Messgerät Messwerte liefert oder eine Insulinpumpe arbeitet, ohne an ein anderes Gerätes angeschlossen zu sein. Es verfügt in diesem Fall auch über eine eigene interne Stromversorgung und ist netzunabhängig betreibbar.

Eine Datenverarbeitungseinrichtung im Rahmen der Erfindung kann beispielsweise ein PDA, ein Datenmanager, ein Kommunikationsadapter (siehe EP 1 762 955 A1) oder ein PC sein, der zum Auslesen, Abspeichern oder Darstellen gespeicherter Daten des Dosiergerätes und des Blutglukosemessgerätes verwendet wird.

Das erfindungsgemäße Dosiergerät umfasst einen integrierten Zeitzähler zum Generieren von relativen Zeitwerten, eine Speichereinheit, in der Datensätze aus Dosiermengen und zugehörigen Zeitwerten abgespeichert werden und eine Vorrichtung zum Übertragen gespeicherter Datensätze an eine Datenverarbeitungseinrichtung. Das Blutglukosemessgerät zum Bestimmen des Blutglukosegehaltes des Körpers umfasst ein integriertes Zeitnormal und eine Speichereinheit, in der Datensätze aus Blutglukosemessungen und zugehörigen Zeitwerten abgespeichert werden, und eine Vorrichtung zum Übertragen gespeicherter Datensätze an eine Datenverarbeitungseinrichtung. Die Vorrichtung zum Übertragen der Datensätze kann dabei in das Blutglukosemessgerät integriert sein, als separates Modul mit diesem verbunden werden oder in die Datenverarbeitungseinrichtung integriert sein.

Dosierungsdaten des Dosiergerätes und Analyseergebnisse des Blutglukosemessgerätes werden zusammen mit Zeitwerten des Zeitzählers des Dosiergerätes und Zeitwerten des Zeitzählers des Blutglukosemessgerätes an die Datenverarbeitungseinrichtung übertragen und dort verarbeitet, wobei die Zeitwerte durch Vergleich mit dem Zeitnormal in korrigierte Zeitwerte umgerechnet werden.

Im Stand der Technik ist eine Vielzahl von zumeist tragbaren Geräten zur Messung des Blutzuckerspiegels bekannt, bei denen eine Blutprobe auf ein Testelement aufgegeben wird, das nachfolgend mit dem Blutglukosemessgerät ausgewertet wird. Derartige Blutglukosemessgeräte besitzen in der Regel einen Speicher, in dem Analyseergebnisse sowie die Zeitpunkte, zu denen die Analysen vorgenommen wurden, abgelegt sind. Im Stand der Technik gibt es weiterhin Systeme, bei denen die Analyseergebnisse eines Blutglukosemessgerätes an eine Auswerteeinheit weitergeleitet werden. Insbesondere bei der Betreuung und Schulung von Diabetikern nehmen derartige Geräte eine zunehmende Bedeutung ein. Auf der Basis von Einzelmessungen kann der Diabetiker lediglich entscheiden, ob Insulin gespritzt werden muss. Durch das Erfassen von Blutzuckermessungen über den Tag und während mehrerer Tage oder Wochen kann der Diabetiker hingegen Informationen darüber gewinnen, wie sein Blutzuckerspiegel durch Nahrungsaufnahme, sportliche Aktivitäten und andere Faktoren beeinflusst wird. Außerdem erhält der Diabetiker durch eine Verlaufskontrolle des Blutzuckerspiegels die wichtige Information, wie sein spezieller Organismus individuell auf die Gabe von Insulin anspricht.

Von der Roche Diagnostics GmbH ist ein Diabetes-Datenmanagement-System im Handel erhältlich. Die mit einem handlichen Blutzuckermessgerät gewonnenen Daten werden bei diesem System an einen PC übertragen, der den Verlauf des Blutzuckerspiegels über die Zeit darstellt und auch Auswertungen ermöglicht, die dem Patienten Anhaltspunkte über die Stärke der vorgenannten Einflußfaktoren geben. Systeme zur Verlaufskontrolle des Blutzuckerspiegels sind so ausgelegt, dass der Benutzer zunächst eine Vielzahl von Messungen durchführt und die Messergebnisse zu einem späteren Zeitpunkt an eine Auswerteeinheit überträgt. Daher ist es notwendig, zusammen mit den Analyseergebnissen auch die Zeitpunkte in dem Blutglukosemessgerät abzuspeichern, zu denen die Analysen durchgeführt wurden.

Da sowohl Dosiergeräte als auch Blutglukosemessgeräte über einen Zeitraum von Monaten oder Jahren zuverlässig arbeiten müssen, ist es erforderlich, in das Gerät entweder eine Uhr mit sehr hoher Ganggenauigkeit einzubauen oder die Möglichkeit vorzusehen, dass die Uhr gestellt werden kann. Einerseits sind Uhren mit hoher Ganggenauigkeit bei wechselnden Umgebungstemperaturen noch relativ teuer. Andererseits ist es für den Benutzer unbequem, ein Stellen der Uhr vornehmen zu müssen. Ein Stellen der Uhr erfordert außerdem zusätzliche Bedienelemente, die in das Gerät integriert werden müssen und es verteuern. Im übrigen ist es auch in vielen Fällen unerwünscht, dass der Diabetiker die Uhr verstellen kann. Häufig wird eine Verlaufskontrolle nämlich durchgeführt, um zu kontrollieren, ob der Diabetiker die vom Arzt verordneten Verhaltensmaßregeln einhält. Ein weiterer Aspekt sind Zeitumstellungen, wie z.B. zwischen Sommer- und Winterzeit sowie beim Wechsel zwischen Zeitzonen. Ein Verstellen der Uhr auf die aktuelle Zeit kann dazu führen, dass eine spätere Zuordnung von Messwerten zu absoluten Zeiten nicht mehr eindeutig möglich ist.

Für solche Fälle kann es daher vorteilhaft sein, Möglichkeiten zum Verstellen der Uhr des Gerätes oder des Zeitzählers zu sperren.

Für eine präzisere und aussagekräftige Verlaufskontrolle einer Diabetesbehandlung und eine Optimierung der Behandlung auf Basis einer genauen Diagnose ist es erforderlich, nicht nur die Verlaufsdaten des Blutglukosemessgerätes zu berücksichtigen, sondern auch die von dem Dosiergerät verabreichten Insulindosierungen genau zu erfassen und mit den gemessenen Werten abzugleichen, um anhand einer hierauf basierten Analyse die Therapie optimieren zu können. Aus diesem Grund weisen die in entsprechenden Systemen verwendeten Dosiergeräte auch eine Speichereinrichtung zum Abspeichern der Verlaufsdaten auf.

Damit ergibt sich für die Auswertung der Daten des Dosiergerätes und des Blutglukosemessgerätes einerseits das Erfordernis, eine Zeiterfassung sowohl der Verlaufsdaten sowohl in dem Dosiergerät als auch dem Blutglukosemessgerät zu ermöglichen als auch die von dem Dosiergerät und dem Blutglukosemessgerät erfassten Daten zeitlich einander zuzuordnen, d.h. zeitlich zu synchronisieren, um auf diese Weise Fehler und Abweichungen in der Zeiterfassung auszugleichen und die Daten mit derselben korrekten Zeitskala auszuwerten. Dabei soll der Aufwand möglichst gering gehalten werden.

Ein System mit einem Dosiergerät, einem Blutglukosemessgerät und einem Computer zum Darstellen von Verlaufsdaten sowie einem das Dosiergerät und das Blutglukosemessgerät mit dem Computer verbindenden Kommunikations-Adapter ist aus der EP 1 762 955 A1 bekannt. Sowohl das Dosiergerät als auch das Blutglukosemessgerät haben ein Zeitnormal, also eine Uhr, und die Speicherung eines Datensatzes in den Geräten schließt die jeweils zugehörige Uhrzeit ein. Dies erfordert einerseits einen hohen Aufwand, und zudem ist das Problem der zeitlichen Synchronisierung und Zuordnung der Verlaufsdaten für den Fall unvermeidlich auftretender Abweichungen der Uhren in den beiden Geräten nicht gelöst. Die Verlaufsdaten werden aus den Geräten unverändert und ohne zeitlichen Abgleich in den Computer bzw. den Kommunikations-Adapter übernommen.

Aus der EP 1 115 435 B1 ist eine Infusionspumpe bekannt, die zum Auslesen von Verlaufsdaten an einen Computer angeschlossen werden kann. Dabei werden die absoluten Uhrzeiten in der Infusionspumpe und dem Computer verglichen und beim Auftreten einer Abweichung wird der Benutzer aufgefordert, die Uhrzeit neu einzustellen (siehe [0039]). Die Uhr in der Infusionspumpe wird dabei durch einen Zähler realisiert, der zum Zeitpunkt der Geräteherstellung gestartet und auf eine absolute Zeit eingestellt wird (siehe [0110]). Die aktuelle Uhrzeit der Insulinpumpe ergibt sich dann aus der Startzeit und der Anzahl der seither verstrichenen Zählpulse. Eine entsprechende Realisierung eines integrierten Zeitnormals bei einem tragbaren Elektrokardiogrammrecorder beschreibt die US 4,653,022.

Aus der DE 197 33 445 A1 und der äquivalenten US 6,216,096 B1 ist ein tragbares, eigenständig ambulant betreibbares Blutglukosemessgerät zum Bestimmen des Blutglukosegehaltes einer Probe eines Körpers bekannt, das einen integrierten Zeitzähler zum Generieren von relativen Zeitwerten, eine Speichereinheit, in der Datensätze aus Blutglukosemessungen und zugehörigen Zeitwerten abgespeichert werden und eine Vorrichtung zum Übertragen gespeicherter Datensätze an eine Datenverarbeitungseinrichtung umfasst.

Aus der WO 2005/043306 ist ein Verfahren zur Aufnahme der Zeiten der Ereignisse einer medizinischen Versorgung und das Bereitstellen dieser Zeiten für die Integration in die Historie der Patientengeschichte bekannt. Hierzu können zwei nicht synchronisierte Zeitquellen von verschiedenen Geräten, jeweils ihre Zeit und charakteristische Informationen der Zeitquelle aufzeichnen. Auf einem Computer wird dann unter Zuhilfenahme dieser Informationen eine Patientengeschichte erstellt.

Die vorliegende Erfindung richtet sich auf ein Konzept des Zeitmanagements bei einem Blutglukosesystem mit einer Kombination aus einem Dosiergerät, insbesondere einem Insulinpen, und einem mit dem Dosiergerät zusammenwirkenden Blutglukosemessgerät, in dem eine Erfassung und Integration von Abgabezeitpunkten, beispielsweise Injektionszeitpunkten, des Dosiergerätes mit Zeitpunkten von Blutzuckermessungen des Blutglukosemessgerätes realisiert wird.

Aufgabe der vorliegenden Erfindung war es, ein solches Blutglukosesystem vorzuschlagen, mit dem die gewonnenen Blutglukosemessergebnisse möglichst exakt den Analysezeitpunkten und den Zeitpunkten der zugehörigen Dosiergaben zugeordnet werden können, und bei dem sowohl teure Präzisionsuhren als auch Bedienelemente zum Einstellen von Uhren weitgehend vermieden werden können.

Erfindungsgemäß wird die Aufgabe durch ein Blutglukosesystem zum Behandeln einer Glukosestoffwechselstörung gelöst, das folgende Merkmale umfasst:
- ein tragbares, eigenständig ambulant betreibbares Dosiergerät zum Abgeben eines medizinischen Wirkstoffs für die Behandlung der Glukosestoffwechselstörung an einen Körper, das einen integrierten Zeitzähler zum Generieren von relativen Zeitwerten, eine Speichereinheit, in der Datensätze aus Dosiermengen und zugehörigen Zeitwerten abgespeichert werden und eine Vorrichtung zum Übertragen gespeicherter Datensätze an eine Datenverarbeitungseinrichtung umfasst,
- ein tragbares, eigenständig ambulant betreibbares Blutglukosemessgerät zum Bestimmen des Blutglukosegehaltes des Körpers, das ein integriertes Zeitnormal und eine Speichereinheit, in der Datensätze aus Blutglukosemessungen und zugehörigen Zeitwerten abgespeichert werden, und eine Vorrichtung zum Übertragen gespeicherter Datensätze an eine Datenverarbeitungseinrichtung umfasst, und
- eine Datenverarbeitungseinrichtung zum Verarbeiten von Datensätzen des Dosiergerätes und des Blutglukosemessgerätes, mit einer Empfangsvorrichtung zum Empfangen der Datensätze von dem Dosiergerät und dem Blutglukosemessgerät sowie einer Recheneinheit zum Umwandeln der Zeitwerte der Datensätze des Dosiergerätes in absolute Zeitwerte auf Basis eines Vergleiches des Zeitwertes des Zeitzählers des Dosiergerätes mit dem Zeitwert eines Zeitnormals,
wobei die Datenverarbeitungseinrichtung dazu ausgebildet ist, die Datensätze des Dosiergerätes und die Datensätze des Blutglukosemessgerätes mittels des Zeitwertes des Zeitnormals durch eine zugeordnete Verknüpfung miteinander zu synchronisieren, dadurch gekennzeichnet, dass das Dosiergerät (3) einen Nadelsensor aufweist, mit dem erfassbar ist, ob sich während des Abgebens von Wirkstoff die verwendete Nadel im Körper befindet, und der Nadelsensor auch als Priming-Sensor verwendet wird, wobei der Bolus als Priming gewertet und der gespeicherte Datensatz entsprechend markiert wird, wenn ein Bolus abgegeben wird, ohne dass die Nadel in den Körper injiziert ist.

Bei der vorliegenden Erfindung wird ein Dosiergerät eingesetzt, das einen Zeitzähler beinhaltet, an den nur geringe Anforderungen bezüglich seiner Ganggenauigkeit gestellt werden müssen. Weiterhin ist das Dosiergerät zum Speichern von Datensätzen aus Dosierabgaben und Zeitwerten ausgestattet. Das Blutglukosesystem beinhaltet auch eine Datenverarbeitungseinrichtung sowie ein Zeitnormal, das sich in der Datenverarbeitungseinrichtung oder dem Blutglukosemessgerät befinden kann. Durch Vergleich des relativen Zeitwertes des Zeitzählers des Dosiergerätes mit der Zeit des Zeitnormals kann der Zeitwert des Zeitzählers in eine absolute Zeit umgerechnet werden und somit können die gespeicherten Dosierverlaufsdaten absoluten Zeitwerten zugeordnet werden. Auf diese Weise können im Dosiergerät preisgünstige Zeitzähler eingesetzt werden und trotzdem ist eine Zuordnung der gespeicherten Verlaufsdaten des Dosiergerätes zu verhältnismäßig exakten Zeiten möglich. Weiterhin werden Fehler durch einen verstellten Zeitzähler vermieden sowie Bedienelemente am Dosiergerät eingespart.

Als Zeitzähler im Dosiergerät können beispielsweise herkömmliche Quarztaktgeber oder elektronische Schieberegister eingesetzt werden. Für die vorliegende Erfindung ist es unerheblich, ob der Zeitzähler eine Uhrzeit oder einfach den Zählerstand eines Schieberegisters liefert. Es ist jedoch wichtig, dass der Zeitzähler keine allzu großen Gangungenauigkeiten aufweisen muss. Die Taktrate des Zeitzählers sollte jedoch möglichst konstant und vorbekannt sein, so dass Differenzen aus verschiedenen Zählerständen in Zeitdifferenzen oder absolute Zeiten umgerechnet werden können. Dies kann nach folgender Formel erfolgen: tA = tD - (nD - nA)/v. tA ist hierbei die absolute Zeit, zu der eine Dosierung A durchgeführt wurde. Zu ihrer Berechnung nach obiger Formel sind notwendig:
- tD: Zeit der Datenübertragung, d.h. eine Zeit zu der Zeitwert des Zeitzählers und Uhrzeit des Zeitnormals verglichen werden können.
- nD: Zählerstand des Zeitzählers bei Vergleich mit dem Zeitnormal (in der Regel bei Datenübertragung).
- nA: Zählerstand des Zeitzählers, der zum Zeitpunkt der Dosisabgabe gespeichert wurde.
- v: Taktrate des Zeitzählers, beispielsweise in Zähleinheiten (Taktpulse, Zählerpulse, Zählschritte) pro Minute.

Die Taktrate (counts per time unit) ist vorzugsweise bekannt und die Datenverarbeitungseinrichtung ist so programmiert, dass sie diese Taktrate berücksichtigt oder die Taktrate wird von dem Dosiergerät an die Datenverarbeitungseinrichtung übertragen.

Die Taktrate kann jedoch auch von der Datenverarbeitungseinrichtung ermittelt werden. Hierzu werden zwei oder mehr zeitlich auseinander liegende Datenübertragungen verwendet: v = (n2 - n1)/(t2 - ti).

Eine Differenz (n2 - n1), die aus zwei Zählerständen gebildet wird, wird durch die zugehörige Differenz der Zeiten des Zeitnormals geteilt. Zugehörig bedeutet, dass t1 und n1 sowie t2 und n2 jeweils zum gleichen realen Zeitpunkt ermittelt wurden. Je länger die Zeiten t1 und t2 auseinander liegen, desto genauer kann die Taktrate v ermittelt werden. Die Taktratenermittlung kann ohne zusätzlichen Aufwand erfolgen, wenn t,n-Wertepaare von einer oder mehreren vorhergehenden Datenübertragungen gespeichert werden und die Taktratenberechnung bei einer erneuten Datenübertragung unter Berücksichtigung gespeicherter Werte sowie des aktuellen t,n-Wertepaares erfolgt. Die vorliegende Erfindung schafft eine Unabhängigkeit in der Ermittlung absoluter Zeitwerte für Dosierabgaben von einem Offset (d.h. einer Verstellung) des Zeitzählers. Der Zeitzähler beginnt vorzugsweise mit dem Einlegen oder Kontaktieren einer Betriebsspannung zu laufen. Es kann auch vorgesehen werden, dass der Benutzer des Dosiergerätes den Zeitzähler stellt. Vorzugsweise kann der Benutzer jedoch keinen Einfluss auf den Zeitwert des Zeitzählers nehmen.

In einer bevorzugten Ausführungsform kann ein Zeitzähler verwendet werden, der beim Anschluss an die Batterie zu laufen beginnt. Der Stand des Zeitzählers wird an eine Umwandlungseinheit weitergeleitet, die aus diesem Wert eine Uhrzeit ermittelt, die auf dem Display dargestellt wird. Über Bedienknöpfe kann dem Benutzer die Möglichkeit gegeben werden, eine Umprogrammierung der Umwandlungseinheit vorzunehmen oder mit anderen Worten, die Uhr zu stellen. Durch den Eingriff des Benutzers bleibt der Wert des Zeitzählers jedoch unberührt, so dass durch ein Stellen der Uhr kein Fehler in der Berechnung der absoluten Zeitpunkte der Dosierabgaben induziert wird. Führt der Benutzer beispielsweise Messungen zur Winterzeit durch und stellt die Uhr danach auf Sommerzeit um, so hat dies keinen Einfluss auf den Zeitzähler und eine eindeutige Zuordnung der Verlaufsdaten zu absoluten Zeiten ist weiterhin möglich.

Vorteilhafterweise kann vorgesehen sein, dass das Dosiergerät eine Umwandlungseinheit besitzt, die Zeitwerte des Zeitzählers in eine Uhrzeit umwandelt und die Umwandlungseinheit vom Benutzer und/oder von der Datenverarbeitungseinrichtung umprogrammiert werden kann, um die Uhrzeit zu korrigieren.

Bei der Verlaufskontrolle von Blutglukosedaten ist es vorteilhaft, eine Vielzahl von Datensätzen aufzunehmen, die Dosierabgaben bzw. Blutglukosewerte und zugehörige Zeitwerte beinhalten. Ein Dosiergerät und entsprechend das Blutglukosemessgerät gemäß der vorliegenden Erfindung benötigen also eine Speichereinheit zum Speichern eines oder mehrerer Datensätze. Handelsübliche Geräte sind in der Regel bereits mit einem derartigen Speicher ausgestattet. Weiterhin besitzen die Geräte auch eine Vorrichtung zum Übertragen gespeicherter Datensätze an die Datenverarbeitungseinrichtung. Bei im Handel befindlichen Systemen wird eine Datenübertragung über Steckverbindungen und Kabel realisiert. Weiterhin ist es auch möglich, eine kabellose Datenübertragung zu realisieren , z.B. über einen Optokoppler (IR) oder per Funk (Bluetooth, RF).

Eine sehr einfache und für den Benutzer bequeme Datenübertragung ist möglich, wenn das Gerät einen Sender enthält, der die Daten an die Datenverarbeitungseinrichtung überträgt, ohne dass eine mechanische Kopplung vorgenommen werden muss. Dadurch wird auch eine galvanische Trennung von Messgerät und Datenverarbeitungseinrichtung erreicht. Dies kann beispielsweise durch eine Infrarotdiode im Gerät und einen entsprechenden Infrarotdetektor in der Datenverarbeitungseinrichtung realisiert werden, wie es von Fernbedienungen für Fernseher bekannt ist. Die Datenübertragung erfolgt bei einer solchen Ausführungsform über moduliertes Licht. Vorteilhaft ist es, wenn die Datenübertragung nicht nur unidirektional vom Gerät zur Datenverarbeitungseinrichtung vorgenommen wird, sondern wenn die Datenverarbeitungseinrichtung ihrerseits auch eine Einheit zum Senden von Daten an das Gerät enthält, so dass eine bidirektionale Kommunikation zwischen den Einheiten möglich ist. Dies kann in günstiger Weise dazu verwendet werden, dass die Datenverarbeitungseinrichtung zunächst das Gerät anspricht und Daten anfordert. Nach erfolgter Datenübertragung kann die Datenverarbeitungseinrichtung den Erhalt quittieren, so dass sichergestellt ist, dass der Datenaustausch vollständig erfolgt ist.

Eine Datenverarbeitungseinrichtung zum Verarbeiten von Verlaufsdaten kann beispielsweise durch einen Personal Computer mit entsprechender Software realisiert werden. Die Datenverarbeitungseinrichtung kann jedoch auch eine handliche Einheit sein, die einem Notebook, Notepad, Palmtop oder dergleichen entspricht. Die Datenverarbeitungseinrichtung beinhaltet eine Recheneinheit (CPU), eine Speichereinheit sowie vorzugsweise ein Display zum Anzeigen von Auswertungsergebnissen.

Die Verarbeitung der Verlaufsdaten durch die Datenverarbeitungseinrichtung kann Berechnungen beinhalten, mit denen die Dosiergaben oder Analyseergebnisse beispielsweise in andere Einheiten umgerechnet werden. Ein wichtiger Aspekt der Verarbeitung der Verlaufsdaten kann auch in ihrer grafischen Darstellung, der Ermittlung von Trends und der Korrelation von Einflussgrößen zu Veränderungen der Analyseergebnisse liegen. Die Verarbeitung der Analyseergebnisse kann beispielsweise in einer Form erfolgen, die einem Diabetiker darüber Aufschluss gibt, wie er sich verhalten muss und welche Insulindosen er injizieren muss, um einen möglichst gleichmäßigen und im Normbereich liegenden Blutzuckerspiegel zu erhalten. Die Datenverarbeitungseinrichtung besitzt eine Vorrichtung zum Empfangen von Datensätzen des Dosiergerätes und des Blutglukosemessgerätes. Wie bereits zusammen mit dem Dosiergerät beschrieben, kann eine derartige Vorrichtung über elektrische Zuleitungen realisiert werden oder auch über Funkempfänger oder optische Empfänger.

Die Datenverarbeitungseinrichtung berücksichtigt bei der Zuordnung der Daten aus dem Dosiergerät und dem Blutglukosemessgerät ein Zeitnormal, das als Referenz dient, um die Zeitwerte des Dosiergerätes in absolute Zeitwerte umzurechnen. Das Zeitnormal kann in dem Blutglukosemessgerät oder in der Datenverarbeitungseinrichtung oder durch eine Kombination von beidem realisiert werden. Demzufolge können in einer ersten vorteilhaften Weiterbildung als Zeitwerte für das Verknüpfen der Datensätze des Dosiergerätes und des Blutglukosemessgerätes die Zeitwerte des Zeitnormals des Blutglukosemessgerätes verwendet werden. Gemäß einer vorteilhaften zweiten Weiterbildung umfasst die Datenverarbeitungseinrichtung ein integriertes Zeitnormal, und als Zeitwerte für das Verknüpfen der Datensätze des Dosiergerätes und des Blutglukosemessgerätes werden die Zeitwerte des Zeitnormals des Blutglukosemessgerätes oder die Zeitwerte des Zeitnormals der Datenverarbeitungseinrichtung verwendet oder es wird eine Kombination von Zeitwerten beider Zeitnormale verwendet

Absolute Zeitwerte im Sinne der Erfindung müssen nicht absolut richtig sein; unter diesem Begriff soll vielmehr verstanden werden, dass im Gegensatz zum Zeitwert des Zeitzählers, der eine Zahl oder eine vollständig falsche Uhrzeit sein kann, auf eine Uhrzeit (und ein Datum) umgerechnet wurde, die um Offsets und gegebenenfalls Gangungenauigkeiten korrigiert wurde. Als Zeitnormal geeignet sind beispielsweise Quarzuhren mit hoher Ganggenauigkeit und Funkuhren. Die in Computern standardmäßig enthaltenen Quarzuhren weisen zwar keine besonders hohe Ganggenauigkeit auf, können jedoch vom Benutzer auf einfache Weise gestellt werden. Das Zeitnormal kann aber auch durch eine per Funk oder über eine Datenverbindung (z.B. über das Internet) von einem Zeitserver bezogen werden.

Eine Stellung des Zeitnormals ist aus mehreren Gründen weniger nachteilig als eine Stellung der im Dosiergerät enthaltenen Zeiterfassung. Zum einen kann die Stellung des Zeitnormals auf einfache und bequeme Weise über eine Tastatur vorgenommen werden. Wenn erforderlich, kann die Datenverarbeitungseinrichtung in der Regel unter Kontrolle eines behandelnden Arztes oder Beraters gestellt werden, so dass Manipulationen vorgebeugt ist. Im übrigen ist es in der Praxis auch so, dass nicht jeder Besitzer eines Analysegerätes über eine eigene Datenverarbeitungseinrichtung verfügt, sondern die Benutzer eines Analysegerätes gehen zu ihrem behandelnden Arzt oder Berater, der über die Datenverarbeitungseinrichtung verfügt. Eine Datenverarbeitungseinrichtung kann daher dazu eingesetzt werden, die Daten vieler verschiedener Blutglukosesysteme zu verarbeiten. Für diese Vielzahl von Blutglukosesystemen ist daher nur ein einziges Zeitnormal, das kontrolliert werden muss, erforderlich.

Die vorliegende Erfindung betrifft weiterhin ein Verfahren zum Verarbeiten von Analyseergebnissen mit einem Blutglukosesystem zum Behandeln einer Glukosestoffwechselstörung, umfassend
- ein tragbares, eigenständig ambulant betreibbares Dosiergerät zum Abgeben eines medizinischen Wirkstoffs für die Behandlung der Glukosestoffwechselstörung an einen Körper, das einen integrierten Zeitzähler zum Generieren von relativen Zeitwerten, eine Speichereinheit, in der Datensätze aus Dosiermengen und zugehörigen Zeitwerten abgespeichert werden und eine Vorrichtung zum Übertragen gespeicherter Datensätze an eine Datenverarbeitungseinrichtung umfasst, und
- ein tragbares, eigenständig ambulant betreibbares Blutglukosemessgerät zum Bestimmen des Blutglukosegehaltes des Körpers, das ein integriertes Zeitnormal und eine Speichereinheit, in der Datensätze aus Blutglukosemessungen und zugehörigen Zeitwerten abgespeichert werden, und eine Vorrichtung zum Übertragen gespeicherter Datensätze an eine Datenverarbeitungseinrichtung umfasst,
- eine Datenverarbeitungseinrichtung zum Verarbeiten von Datensätzen des Dosiergerätes und des Blutglukosemessgerätes, mit einer Empfangsvorrichtung zum Empfangen der Datensätze von dem Dosiergerät und dem Blutglukosemessgerät sowie einer Recheneinheit zum Umwandeln der Zeitwerte der Datensätze des Dosiergerätes in absolute Zeitwerte auf Basis eines Vergleiches des Zeitwertes des Zeitzählers des Dosiergerätes mit dem Zeitwert eines Zeitnormals,
wobei die Datenverarbeitungseinrichtung dazu ausgebildet ist, die Datensätze des Dosiergerätes und die Datensätze des Blutglukosemessgerätes mittels des Zeitwertes des Zeitnormals durch eine zugeordnete Verknüpfung miteinander zu synchronisieren,
das die folgenden Schritte umfasst:
- Abspeichern eines oder mehrerer Datensätze in dem Dosiergerät von mit dem Dosiergerät durchgeführten Abgaben eines medizinischen Wirkstoffs für die Behandlung der Glukosestoffwechselstörung, beinhaltend Dosiermengen sowie Zeitwerte, die mittels des integrierten Zeitzählers zum Zeitpunkt der jeweiligen Abgabe gewonnen werden,
- Abspeichern eines oder mehrerer Datensätze in dem Blutglukosemessgerät von mit dem Blutglukosemessgerät durchgeführten Blutglukosemessungen, beinhaltend Analyseergebnisse sowie Zeitwerte, die mittels des integrierten Zeitnormals zum Zeitpunkt der jeweiligen Messung gewonnen werden,
- Übertragen eines oder mehrerer Datensätze des Blutglukosemessgerätes und des Dosiergerätes an die Datenverarbeitungseinrichtung sowie Übergabe des aktuellen Zeitwertes des Zeitzählers des Dosiergerätes an die Datenverarbeitungseinrichtung,
- Berechnen der absoluten Zeitwerte, zu denen der Wirkstoff von dem Dosiergerät abgegeben wurde, mittels der Datenverarbeitungseinrichtung aus den Zeitwerten des Datensatzes des Dosiergerätes durch Vergleich des aktuellen Zeitwertes des Zeitzählers des Dosiergerätes mit dem exakten Zeitwert eines Zeitnormals, wobei die Datensätze des Dosiergerätes und die Datensätze des Blutglukosemessgerätes mittels des Zeitwertes des Zeitnormals durch eine zugeordnete Verknüpfung miteinander synchronisiert werden, und
- Verarbeiten der Dosiermengen und Analyseergebnisse auf Basis der berechneten Zeitpunkte, dadurch gekennzeichnet, dass das Dosiergerät (3) einen Nadelsensor aufweist, der erfasst, ob sich während des Abgebens von Wirkstoff die verwendete Nadel im Körper befindet, und der Nadelsensor auch als Priming-Sensor verwendet wird, wobei der Bolus als Priming gewertet und der gespeicherte Datensatz entsprechend markiert wird, wenn ein Bolus abgegeben wird, ohne dass die Nadel in den Körper injiziert ist.
Unter dem Schritt des Verarbeitens von Dosiermengen und Analyseergebnissen kann in weitestem Sinne das Abspeichern, Übertragen oder Darstellen der synchronisierten Datensätze des Dosiergerätes und des Blutglukosemessgerätes verstanden werden. Die Funktionsweise des erfindungsgemäßen Verfahrens wird anhand des folgenden Beispieles erläutert.

Beim Kontaktieren des Zeitzählers mit einer Betriebsspannung beginnt dieser mit konstanter und vorbekannter Taktrate zu laufen. Vom Benutzer wird mit dem Dosiergerät zum Zeitpunkt T1 eine erste Dosierung vorgenommen und ein entsprechender Datensatz abgespeichert, der die abgegebene Dosis und den zugehörigen Zeitzählerwert umfasst. Einige Zeit später begibt sich der Benutzer zu seinem Arzt, wo eine Übertragung des Datensatzes zum Zeitpunkt T2 an die Datenverarbeitungseinrichtung vorgenommen wird. Zusammen mit dem Dosiswert und dem zugehörigen Zeitwert wird auch der aktuelle Zeitwert des Zeitzählers mit von dem Dosiergerät an die Datenverarbeitungseinrichtung übertragen. Zusätzlich werden von der Danteverarbeitungseinrichtung auch Datensätze des Blutglukosemessgerätes ausgelesen.

Die Datenverarbeitungseinrichtung bildet die Differenz zwischen dem aktuellen Zeitwert des Zeitzählers des Dosiergerätes und dem zugehörigen relativen Zeitwert des Zeitnormals. Bei einer nachfolgenden Auswertung der Verlaufsdaten des Dosiergerätes werden die relativen Zeitwerte der Datensätze durch Subtraktion der Zeitdifferenz in absolute Zeitwerte umgerechnet. Es ist klar, dass die Zeitwerte von Zeitzähler und Zeitnormal, die zur Berechnung der Differenz herangezogen werden, den gleichen realen Zeitpunkt repräsentieren müssen. Daher wird für die Differenzbildung vorzugsweise der aktuelle Zeitwert des Zeitzählers übertragen und der im Zeitpunkt der Übertragung vorliegende Zeitwert des Zeitnormals herangezogen. Aus dem Zeitwert des Zeitnormals zu einem bestimmten Zeitpunkt und dem Zeitwert des Zeitzählers zum gleichen Zeitpunkt wird eine Zeitdifferenz ermittelt und die Zeitwerte der Datensätze werden durch Addition der Zeitdifferenz in absolute Zeitwerte umgerechnet.

Ein weiterer vorteilhafter Aspekt der vorliegenden Erfindung ist es, dass mit einer Datenübertragung eine Synchronisation mit den von dem Blutglukosemessgerät heruntergeladenen Datensätzen erfolgen kann, das heißt das auch diese Daten zusammen mit den Daten des Dosiergerätes auf eine einheitliche gemeinsame genaue absolute Zeit umgerechnet und zueinander zugeordnet werden. Ferner kann der Zeitzähler in dem Dosiergerät gestellt werden oder der Stand des Zeitzählers und die zugehörige Zeit des Zeitnormals wird gespeichert, so dass dieses Datenpaar als neue Berechnungsbasis für eine Umwandlung von Zeitwerten in absolute Zeiten dienen kann. Hierdurch haben Offsets des Zeitzählers sowie Gangungenauigkeiten, die vor der Synchronisation aufgelaufen sind, keinen Einfluss mehr auf Zeitberechnungen, die nach der Synchronisation erfolgen.

Die Vorteile eines erfindungsgemäßen Systems lassen sich anhand der nachstehenden Tabelle besonders deutlich erkennen:

**Tabelle 1**

| | Zeitzähler | Zeitnormal | Zeile |
|---|---|---|---|
| Werkseitige Einstellung | 15.06.2002 | 15.06.2002 | 1 |
| | 9:30:00 | 9:30:00 | |
| Dosisabgabe I | 15.07.2002 | 15.07.2002 | 2 |
| | 8:40:00 | 8:45:00 | |
| Übertragung | 17.07.2002 | 17.07.2002 | 3 |
| | 10:25:00 | 10:30:20 | |
| Übertragung | 15.01.2007 | 15.01.2007 | 4 |
| | 9:30:00 | 15:00:00 | |
| Dosisabgabe II | 16.01.2007 | 16.01.2007 | 5 |
| | 9:30:00 | 15:00:10 | |
| Übertragung | 22.01.2007 | 22.01.2007 | 6 |
| | 10:00:00 | 15:31:10 | |

Für die obige Tabelle wurde ein Zeitzähler eines Dosiergerätes zugrunde gelegt, der etwa 10 sec/Tag nachgeht. Die Zeile 2 der Tabelle zeigt, dass bereits bei einer Dosisabgabe zwei Monate nach Stellung des Zeitzählers ein Gangunterschied zu dem die richtige Absolutzeit angegebenen Zeitnormal (in dem Blutglukosemessgerät oder der Datenverarbeitungseinrichtung) von 5 Minuten auftritt. Geht der Patient zwei Tage später zu seinem Arzt und lässt eine Übertragung der Datensätze in Verbindung mit den Datensätzen der Datensätze des Blutglukosemessgerätes vornehmen, so erkennt das erfindungsgemäße System eine Zeitdifferenz von Zeitzähler zu Zeitnormal von 5 Minuten und 20 Sekunden. Bereits eine einfache Addition dieser Differenz zu dem Zeitwert für die Dosisabgabe I reduziert den Gangunterschied von 5 Minuten auf einen von 20 Sekunden. Bei Anwendung des Systems zur Verlaufskontrolle von Blutzuckerspiegeln ist ein Gangunterschied von 5 Minuten bei den meisten Auswertungen jedoch von untergeordneter Bedeutung. Daher wurde in Tabelle 1 eine entsprechende Betrachtung für eine Messung fünf Jahre nach Einstellung des Zeitzählers durchgeführt. Zeile 4 der Tabelle 1 zeigt, dass bei einer Dosisabgabe am 15.01.2007 bereits ein Gangunterschied von vier Stunden zwischen den Zeitwerten der Datensätze des Dosiergerätes und den Zeitwerten der Datensätze des Blutglukosemessgerätes vorliegt. Würden Dosierwerte und Analyseergebnisse im Jahre 2007 ohne eine Korrektur ausgewertet werden, so würde eine Verlaufskontrolle des Blutzuckerspiegels vollständig fehl laufen, da ein Blutzuckerspiegel aus der Mittagszeit als ein morgendlicher Blutzuckerspiegel interpretiert würde.

Eine Dosisabgabe gemäß Zeile 5 der Tabelle 1 kann auf einfache Weise hinsichtlich der Zeitwerte korrigiert werden, wenn bei dem Übertragen der Datensätze, d.h. der nächsten Übertragung gespeicherter Datensätze von dem Dosiergerät an die Datenverarbeitungseinrichtung die Zeitdifferenz zwischen dem Zeitzähler des Dosiergerätes und dem Zeitnormal ermittelt und die ermittelte Zeitdifferenz gleich oder zu einem späteren Zeitpunkt zum Korrigieren der Zeitwerte der Datensätze des Dosiergerätes verwendet wird. Beispielsweise kann bei einer ersten Datenübertragung eine erste Zeitdifferenz zwischen Zeitnormal und Zeitzähler ermittelt und bei einer darauffolgenden zweiten Datenübertragung eine entsprechende zweite Zeitdifferenz und zwischen den Datenübertragungen liegende Zeitwerte durch Interpolation zwischen erster und zweiter Zeitdifferenz und Addition der interpolierten Zeitdifferenz zu dem Zeitwert der Dosisabgabe ermittelt werden. Wird beispielsweise am 22.01.2007 eine Übertragung vorgenommen, so errechnet sich die Zeitdifferenz zu 5 Stunden 31 Minuten und 10 Sekunden. Bei Addition dieser Differenz zu dem Wert des Zeitzählers am 16.01.2007 (siehe Spalte 5) kann die Ungenauigkeit auf 1 Minute reduziert werden, was für normale Verlaufskontrollen gut hinnehmbar ist.

Vorteilhaft kann für Auswertungen zu einem bestimmten Zeitpunkt eine Korrektur des vom Zeitzähler ermittelten Zeitwertes durch Interpolation von Gangungenauigkeiten ermittelt werden, die bei Übertragung vor der Messung und nach der Messung gefunden wurden. Aus den in den Zeilen 4 und 6 dargestellten Daten kann beispielsweise ermittelt werden, dass innerhalb von 7 Tagen eine Gangungenauigkeit von 70 Sekunden aufgetreten ist. Dementsprechend kann zurückgeschlossen werden, dass zu der am 15.01.2007 durch Differenzbildung ermittelten Gangungenauigkeit bis zum Datum der Auswertung (Zeile 5) weitere 10 Sekunden Gangunterschied hinzugekommen sind. Da in der Praxis Gangunterschiede jedoch keine Materialkonstanten sind, sondern durch Temperaturunterschiede und andere Einflussfaktoren in unterschiedlichem Ausmaß hervorgerufen werden, ist die durch Interpolation erzielbare Genauigkeit begrenzt.

Das Dosiergerät hat eine relative Zeit und das Blutglukosemessgerät hat eine absolute Zeit. Die Datenverarbeitungseinrichtung hat entweder keine Zeitanbindung oder kann auch über ein Zeitnormal verfügen. Wenn die Datenverarbeitungseinrichtung keine Zeitanbindung hat, liest sie die Zeit aus dem Blutglukosemessgerät aus, rechnet die Daten von dem Dosiergerät damit um und erzeugt so einen gemeinsamen Datensatz aus den Daten des Dosiergerätes und des Blutzuckermessgerätes für die Darstellung. Falls die Datenverarbeitungseinrichtung eine Zeitanbindung oder ein Zeitnormal hat, kann dabei alternativ oder zusätzlich auch das Zeitnormal der Datenverarbeitungseinrichtung verwendet werden.

Die Insulinabgabe mit dem Dosiergerät kann durch den Benutzer zu einem beliebigen Zeitpunkt ausgelöst oder auch mit einer festen Frequenz, zum Beispiel einmal pro Minute, erfolgen. Die Dosiermengen werden in dieser festen Reihenfolge in dem Speicher des Dosiergerätes abgelegt und der Speicherpointer wird um eins erhöht. Beim Auslesen der Daten durch die Datenverarbeitungseinrichtung werden nur der aktuelle Speicherpointer, der zugehörige Messwert und die davor liegenden Messwerte, aber ohne deren Pointer übertragen. Zum Rekonstruieren des Verlaufs ordnet die Datenverarbeitungseinrichtung dem aktuellen Messwert den absoluten Zeitpunkt zu, nämlich den Zeitpunkt der Datenübertragung, unter Zugrundelegung des Zeitnormals des Blutglukosemessgerätes, der Datenverarbeitungseinrichtung oder einer Kombination von beiden, und legt den Dosierwert mit der absoluten Zeit im Speicher der Datenverarbeitungseinrichtung ab. Die vorangegangenen Dosierwerte werden ohne Zeiten in dem Dosiergerät abgespeichert. Der Zeitbezug zum Zeitpunkt der Datenübertragung an die Datenverarbeitungseinrichtung wird wiederum über eine Zeitregel und den Speicherpointer in dem Dosiergerät hergestellt. Hierdurch wird Speicherplatz eingespart. Voraussetzung, dass einem bestimmten Datensatz eine Absolutzeit zugeordnet werden kann, ist, dass die Datenverarbeitungseinrichtung in dem entsprechenden Zeitintervall mindestens einmal auf das Dosiergerät zugreift und die Daten übertragen werden.

Das Dosiergerät verfügt über eine automatische Datenerhebung und Datenspeicherung mit einer Zeitmarkierung auf relativer Zeitbasis. Dies kann einerseits in einem fest vorgegebenen zeitlichen Abstand der einzelnen Dosier- oder Messpunkte erfolgen oder vom Benutzer aufgrund seiner subjektiven Befindlichkeit ausgelöst werden.

Beispielsweise kann ein Quarz ein Zeitsignal erzeugen, aus dem ein Mikrocontroller ein genaues Zeitintervall ableitet. Bei einem auf diese Weise realisierten Zeitzähler wird beispielsweise jede Minute ein Datensatz erzeugt und fortlaufend im Speicher abgelegt. Eine Konvention in dem System macht über die Kenntnis der Speicheradresse dann die in der Datenverarbeitungseinrichtung erfolgende Zeitzuordnung möglich. Ein Unterschied zu einem Blutglukoserecorder, d.h. einem Blutglukosemessgerät mit Verlaufsmessung, das auch über eine Zeitsteuerung verfügt, besteht darin, dass bei einem solchen in vorgegebenen Zeitabständen messenden Blutglukoserecorder die Zeitpunkte der Blutglukosemessung mittels des Zeitzählers ausgelöst werden, d.h. der Zeitzähler ist der die Messung auslösende Trigger und über die Speicherplatzadresse wird die zeitliche Zuordnung der Messung ermöglicht. Bei einem erfindungsgemäßen Dosiergerät dagegen führt der Benutzer zu irgendeinem Zeitpunkt eine Abgabe von Insulin durch und dieser Dosierabgabe wird dann ein Zählerstand zugeordnet. Das Auslösen, d.h. das Triggern erfolgt somit durch den Benutzer und der Zeitzähler ermöglicht die zeitliche Zuordnung des Dosisabgabe zu einer Zeit.

Bei einem Blutglukoserecorder wird also mit einer festen Messfrequenz von zum Beispiel 1 Hz gemessen und die Messwerte werden in einer festen Reihenfolge im Speicher abgelegt. Beim Auslesen braucht nur der Speicherplatzindex und der Messwert übertragen zu werden, um den Verlauf rekonstruieren zu können. Ein Zeitzähler im Sinne der Erfindung ist somit bei einem Blutglukosemessgerät nicht erforderlich. Bei einem erfindungsgemäßen Dosiergerät dagegen sind Zeitpunkt und Häufigkeit der Dosierabgaben unbekannt. Daher muss zumindest eine relative zeitliche Zuordnung der Dosierabgaben in den Datensätzen mit abgespeichert werden, um später eine chronologische Verlaufszuordnung der Historie zu ermöglichen. Hierzu kann ein Zeitnormal oder zur Vereinfachung des apparativen Aufwands ein erfindungsgemäßer Zeitzähler verwendet werden.

Der Zeitzähler dient zur zeitlichen Zuordnung der im Dosiergerät gespeicherten Daten (Boli) zu den Blutglukosemesswerten des Blutglukosemessgerätes, wenn die Daten des Dosiergerätes und des Blutglukosemessgerätes in der Datenverarbeitungseinrichtung oder einem an die Datenverarbeitungseinrichtung angeschlossenen Gerät gemeinsam in einem Grafen oder nebeneinander in mehreren Grafen dargestellt werden, wobei die relative zeitliche Zuordnung der Daten aus dem Dosiergerät zu den Daten aus dem Blutglukosemessgerät ermittelt und berücksichtigt werden muss. Erfindungsgemäß ist in dem Dosiergerät kein Zeitnormal, sondern ein Zeitzähler integriert. Der Zeitzähler dient zur relativen Zeiterfassung. Hierzu ist ein Zeitgeber bzw. ein Zähler in das Dosiergerät integriert, der in einem festen Takt hoch zählt oder herunter zählt. Zu jedem abgegebenen Bolus wird der zugehörige Zählerstand des Zeitzählers gespeichert. Beim Einlesen der von dem Dosiergerät und dem Blutglukosemessgerät gespeicherten Verlaufdaten wird die aktuelle Zeit des verwendeten Zeitnormals, d.h. des Blutglukosemessgerätes, der Datenverarbeitungseinrichtung oder eine Kombination von beiden als Startzeit genommen und dem aktuellen Zählerstand zugeordnet. Von diesem Zählerstand ausgehend werden dann zeitlich zurückliegenden gespeicherten Boli absolute Zeiten zugeordnet.

Vorteile dieser Vorgehensweise sind, dass keine Zeitsynchronisation zwischen dem Dosiergerät und der Datenverarbeitungseinrichtung erforderlich ist und an dem Dosiergerät keine Zeit eingesellt werden muss.

Die Verlaufsdaten des Dosiergerätes können auch nur mit einer relativen Zeitachse von der Datenverarbeitungseinrichtung in grafischer Form dargestellt werden; andernfalls wird der Zeitreferenzpunkt von dem Benutzer oder dem Zeitnormal, also dem Blutglukosemessgerät oder der Datenverarbeitungseinrichtung vorgegeben.

Auf dem Dosiergerät kann die seit dem letzten Bolus bzw. den letzten Boli verstrichene relative Zeitdauer angezeigt werden. Eine solche relative Zeitangabe wird den meisten Benutzern genügen oder sogar erwünschter sein als eine absolute Angabe der Zeit des letzten Bolus, da in diesem Fall der Benutzer erst noch mittels der aktuellen Uhrzeit die verstrichene Zeitdauer berechnen muss.

Ein weiterer Vorteil besteht darin, dass selbst wenn die Zeit des Dosiergerätes unrichtig ist, die relative Zuordnung der Daten des Dosiergerätes zu denen des Blutglukosemessgerätes korrekt bleibt und keine Fehlinterpretation der kombinierten Daten erfolgt, zumindest solange in dem Blutglukosemessgerät keine Zeitverstellungen vorgenommen werden, die für den Fall, dass sie nicht markiert werden, unerkannt bleiben.

Die für Insulinpens vorgeschriebene Begrenzung der Laufzeit auf zwei Jahre kann durch den Zeitzähler ebenfalls überwacht und erfasst werden, ohne dass hierfür eine absolute Uhr erforderlich ist.

Des Weiteren kann der Zeitzähler in dem Dosiergerät auch zum Erzeugen und Darstellen einer aktuellen Uhrzeit an dem Dosiergerät dienen. Hierzu wird bei der Herstellung des Dosiergerätes bei einem bestimmten Zählerstand des Zeitzählers, beispielsweise bei dem Zählerstand Null, die aktuelle Absolutzeit abgelegt; aus dem Zählerstand kann dann die Absolutzeit für andere spätere Zeitpunkte errechnet werden. Dies kann für verschiedene Anwendungen zweckmäßig sein, beispielsweise für folgende: 1) Zum Darstellen der aktuellen Uhrzeit in der Anzeige des Dosiergerätes. 2) Beim Übertragen der Datensätze aus dem Dosiergerät an die Datenverarbeitungseinrichtung, ohne dass ein Zeitnormal (von dem Blutzuckermessgerät oder der Datenverarbeitungseinrichtung) zur Verfügung steht, kann eine zumindest orientierend richtige Zeitachse definiert werden. 3) Die Daten des Dosiergerätes und des Blutglukosemessgerätes können synchronisiert werden und es kann geprüft werden, ob die aktuelle Zeit an beiden Geräten übereinstimmt. 4) Bei jeder Kommunikation mit einer Datenverarbeitungseinrichtung, zum Beispiel beim Übertragen der Daten an die Datenverarbeitungseinrichtung, kann das Dosiergerät die absolute Zeit der Datenverarbeitungseinrichtung (zum Beispiel die PC-Zeit) abfragen und so die zeitliche Zuordnung zu dem Zählerstand des Zeitzählers in dem Dosiergerät wieder kalibrieren. 5) Die weltweite zeitliche Synchronisation, wobei der Zeitzähler bei seinem Einbau in das Dosiergerät auf eine weltweit synchronisierte Standardzeit kalibriert wird und beim Auslesen der Zeitwert wieder in die weltweit synchronisierte Standardzeit und von dort in die lokale absolute Zeit umgerechnet wird.

Die grundsätzlichen Vorteile einer in das Dosiergerät integrierten Zeiterfassung sind folgende:
- Die Dauer einer Injektion kann erfasst werden. Diese Erfassung in dem Datensatz des Dosiergerätes erfolgt beispielsweise in der Weise, dass in dem Datensatz zu zugehörigen Zeitwerten jeweils der Beginn und das Ende einer Dosierabgabe vermerkt werden. Beim Auslesen des Datensatzes in dem Dosiergerät zum Anzeigen mit dem Dosiergerät oder beim Auslesen des Dosiergerätes mit einer Datenverarbeitungseinrichtung stehen dann nicht nur die jeweiligen Injektionszeitpunkte zur Auswertung zur Verfügung, sondern auch die zugehörigen Injektionsdauern. Je nach Auslegung des Dosiergerätes kann die Injektionsdauer eine Information über die Injektionsgeschwindigkeit und/oder die Dosiermenge beinhalten. Ferner kann auf der Anzeige des Dosiergerätes dargestellt werden, wie langsam die Dosierung erfolgen soll, oder bei einer Datenauswertung kann eine zu schnelle Injektion, bei der das Insulin an der Einstichstelle vorbeispritzt oder wieder austritt, als mögliche Fehlerquelle erkannt werden.
- Aus der Insulinmenge und der Injektionszeit kann die Injektionsgeschwindigkeit, der so genannte Flow bestimmt werden. Mit dieser Information kann eine empfohlene mengenabhängige Injektionszeit auf der Anzeige dargestellt werden, beispielsweise durch einen Countdown, oder bei einer Datenauswertung kann eine zu schnell durchgeführte Injektion als mögliche Fehlerquelle erkannt werden.
- Es ist ein zusätzlicher Nadelsensor eingebaut, mit dem erfasst wird, ob sich während des Abgebens von Wirkstoff die verwendete Nadel im Körper befindet, z.B. ein mechanischer Taster oder eine elektrische Leitfähigkeitsmessung der Nadel, kann auf der Anzeige des Dosiergerätes angezeigt werden, wie lange die Nadel nach der Injektion in dem Körper bleiben soll. Empfohlen ist hierfür eine Zeitdauer von ca. 10 sec. In dem von dem Dosiergerät gespeicherten Datensatz kann erfasst bzw. abgespeichert werden, wie lange die Nadel tatsächlich in dem Körper verblieben ist, um bei der Datenauswertung Abweichungen als mögliche Fehlerursache zu erkennen. Ein solcher Nadelsensor wird auch als Priming-Sensor verwendet werden; wenn ein Bolus abgegeben wird, ohne dass die Nadel in den Körper injiziert ist, wird der Bolus als Priming gewertet und wird nicht als letzter Bolus auf der Anzeige des Dosiergerätes oder bei der grafischen Darstellung mittels der Datenverarbeitungseinrichtung angezeigt. Der gespeicherte Datensatz wird entsprechend markiert.

Mit Priming wird die Überprüfung des Insulinflusses zur Vorbereitung des Insulinpens vor der Injektion bezeichnet. Bei Lagerung einer angebrochenen Insulinkartusche mit aufgesteckter Nadelspitze und vor der erstmaligen Verwendung einer neuen Insulinkartusche können sich Luftblasen in der Kartusche und/oder der Nadelspitze befinden, wodurch die am Insulinpen eingestellte Insulindosis eventuell nicht vollständig abgegeben wird. Während des Priming wird der Insulinpen mit der Injektionsnadel nach oben gehalten. Mit dem Finger wird einige Male vorsichtig gegen die Insulinpatrone geklopft, damit eventuell vorhandene Luftblasen nach oben aufsteigen. Die Dosisanzeige wird bei einer neuen Patrone beispielsweise auf vier Einheiten und bei einer angebrochenen Patrone auf eine Einheit eingestellt. Die eingestellte Dosis wird senkrecht in die Luft abgegeben. Dabei muss aus der Nadelspitze ein Insulintropfen austreten, d.h. die Luft ist vollständig entfernt. Ist dies nicht der Fall, wird die Dosisabgabe wiederholt, bis aus der Nadelspitze ein Insulintropfen austritt.
- Ferner kann der Kartuschenwechsel interpretiert und so die Kartuschenverwendungsdauer, beispielsweise die mittlere Dauer, die Abweichungen hiervon usw., angezeigt, gespeichert und ausgewertet werden.
Erfindungsgemäße Verfahren zum Auswerten von Analyseergebnissen können verbessert werden, indem zusammen mit den übertragenen Datensätzen ein oder mehrere Prüfsummen an die Datenverarbeitungseinrichtung übermittelt werden, die eine Kontrolle zulassen, ob die Datenübertragung fehlerfrei erfolgt ist. Weiterhin ist es vorteilhaft, wenn eine gerätespezifische Kennung des Dosiergerätes und des Blutglukosemessgerätes mit übertragen wird, so dass die Datenverarbeitungseinrichtung zwischen verschiedenen Analysegeräten unterscheiden kann. Bereits vorstehend wurde beschrieben, dass es in der Praxis häufig der Fall sein wird, dass eine Vielzahl von Benutzern mit ihren eigenen Dosiergeräten und Blutglukosemessgeräten zu einem Arzt oder Berater gehen, um dort eine Auswertung mit einer Datenverarbeitungseinrichtung vornehmen zu lassen. Bei Verwendung einer Kennung der jeweiligen Geräte können Verwechslungen vermieden werden.

Die Erfindung wird nachfolgend anhand eines in der Figur dargestellten Ausführungsbeispiels näher erläutert. Die darin beschriebenen Besonderheiten können einzeln oder in Kombination miteinander eingesetzt werden, um bevorzugte Ausgestaltungen der Erfindung zu schaffen.

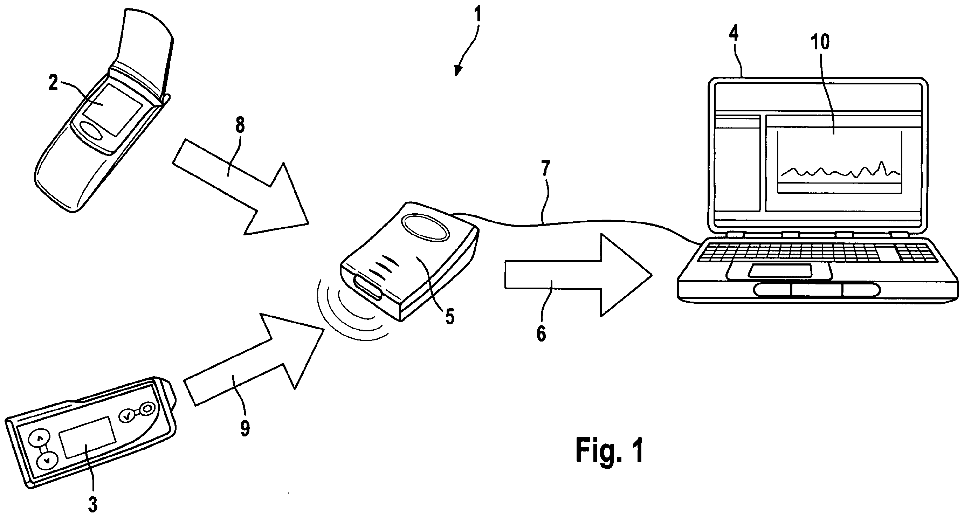

Die Fig. 1 zeigt Komponenten eines erfindungsgemäßen Blutglukosesystems 1, umfassend ein Dosiergerät 2 in Form einer Insulinpumpe (oder alternativ beispielsweise ein Insulinpen), ein Blutglukosemessgerät bzw. Blutglukosemeter 3 sowie eine Datenverarbeitungseinrichtung 4. Als zusätzliches Gerät ist ein Kommunikations-Adapter 5 vorgesehen, an den das Dosiergerät 2 und das Blutglukosemessgerät 3 angeschlossen werden. Die Funktionsweise des optionalen Kommunikations-Adapters 5 ist in der EP 1 762 955 A1 beschrieben. Das Dosiergerät 2 und das Blutglukosemessgerät 3 sind tragbare, netzunabhängig ambulant betreibbare medizinische Geräte, die von einem Benutzer in Kombination miteinander verwendet werden. Beide Geräte weisen eine Schnittstelle zum Senden und/oder Empfangen von Daten auf, die beispielsweise als Infrarot-Schnittstelle ausgebildet ist.

Das Blutglukosemessgerät 2 arbeitet mit Testelementen, die in einen Schacht des Gerätes eingeschoben werden. Das Blutglukosemessgerät 2 besitzt weiterhin Bedienelemente sowie eine Anzeige zum Darstellen von Analyseergebnissen. Von besonderer Bedeutung für die vorliegende Erfindung ist eine Speichereinheit, die in das Blutglukosemessgerät 2 integriert ist und zum Speichern von Datensätzen dient, die Analyseergebnisse und zugehörige Zeitwerte beinhalten. Außerdem besitzt das Blutglukosemessgerät 2 ein Zeitnormal, das zu Analysezeitpunkten gehörende Zeitwerte an die Speichereinheit weitergibt. Zur Kommunikation mit der Datenverarbeitungseinrichtung 4 besitzt das Blutglukosemessgerät 2 weiterhin eine drahtgebundene oder drahtlose Schnittstelle.

Das Dosiergerät 3 kann Insulindosen an einen Körper abgeben. Es besitzt weiterhin Bedienelemente sowie eine Anzeige zum Darstellen von Funktionsparametern. Von besonderer Bedeutung für die vorliegende Erfindung ist eine Speichereinheit, die in das Dosiergerät 3 integriert ist und zum Speichern von Datensätzen dient, die Dosierabgaben und zugehörige Zeitwerte beinhalten. Außerdem besitzt das Blutglukosemessgerät 2 einen Zeitzähler, das zu Dosierzeitpunkten gehörende Zeitwerte an die Speichereinheit weitergibt. Zur Kommunikation mit der Datenverarbeitungseinrichtung 4 besitzt das Dosiergerät 3 weiterhin eine drahtgebundene oder drahtlose Schnittstelle.

Der Kommunikations-Adapter 5 ist für die drahtgebundene Datenübertragung 6 mit einem Übertragungskabel 7 an die Datenverarbeitungseinrichtung 4 angeschlossen. Die Datenverarbeitungseinrichtung 4 ist ein Computer und kann beispielsweise ein Personal-Computer, ein Laptop, ein Handheld-Computer oder der Computer in einem Internet-Cafe, in einer Arztpraxis, zu Hause oder in einer Apotheke, wohin sich der Benutzer des Systems 1 nur mit seinen Geräten 2, 3 oder mit seinen Geräten 2, 3 und einem Kommunikations-Adapter 5 begibt, sein. Die Datenverarbeitungseinrichtung 4 beinhaltet neben einer Rechnereinheit optional ein Zeitnormal.

Zum Auslesen der Daten der Geräte 2, 3 mittels der Datenübertragungen 8, 9 und deren Darstellung auf dem Monitor 10 des Computers 4 wird zunächst der Kommunikations-Adapter 5 mit dem Kabel 7 an den Computer 4 angeschlossen. Der Kommunikations-Adapter 5 kann aber auch im Computer integriert sein und die Datenübertragung kann direkt von den Geräten 2, 3 an den Computer 4 erfolgen.

Das Dosiergerät 3 hat keine Uhr, also keine absolute Zeit, sondern nur einen Zeitzähler. Bei dem Zeitzähler handelt es sich um einen Taktgeber, also ein Zeitdifferenznormal, mit einem Speicher in Form einer Tabelle. Die in der Tabelle abgespeicherten Datensätze umfassen jeweils die Anzahl der Ticks (Takte) des Zeitzählers, die seit der letzten Insulinabgabe verstrichen sind, und die abgegebene Insulinmenge, aber nicht den Zeitpunkt, zu dem die Insulinabgabe erfolgte.

Diese Daten werden aus dem Dosiergerät 3 in das Datenverarbeitungsgerät 4 heruntergeladen. Dieses Datenverarbeitungsgerät 4 hat ein Zeitnormal (eine Uhr) zur Verfügung, und zwar entweder ein integriertes oder ein Zeitnormal von dem Blutglukosemessgerät 2 und verfügt somit über die absolute Zeit. Beim Download der Daten von dem Dosiergerät 2 wird der Download-Zeitpunkt, der dem Datenmanager als absolute Zeit bekannt ist, verwendet, um aus der gespeicherten Anzahl von Ticks (Counts) die absoluten Zeitpunkte, zu denen die Insulinabgaben erfolgten, rückzurechnen. Es kann aber auch genügen, wenn der Download-Zeitpunkt gespeichert wird, beispielsweise in dem Kommunikations-Adapter 7, und die Rückrechnung auf die Dosierungszeitpunkte erst im Datenverarbeitungsgerät 4 erfolgt.

Falls sich bei der Datenverarbeitung herausstellen sollte, dass die Uhr in dem Datenmanager von der tatsächlichen Zeit abweicht, beispielsweise durch einen Vergleich mit der Zeit des PCs 4, die in vielen Fällen über das Internet mit der Zeit synchronisiert wird, kann diese systematische Abweichung der Uhr des Datenmanagers leicht durch eine entsprechende Korrekturverschiebung berücksichtigt werden.

Ein Vorteil des erfindungsgemäßen Zeitzählers in dem Dosiergerät 3 besteht darin, dass im Unterschied zum Stand der Technik keine Synchronisation zwischen einer Uhr des Dosiergerät 3 und einer Uhr des Datenmanagers erfolgen muss, wobei offen bliebe, welche der zwei Uhren im Falle der Abweichung der Vorrang hat oder wie die Abweichung behandelt wird. Ferner kann durch den späteren optionalen Abgleich mit einer Weltzeit, beispielsweise über den PC 4, auch eine absolute Kalibrierung in einfacher Weise erfolgen.

### Bezugszeichenliste

- 1: Blutglukosesystem
- 2: Blutglukosemeter
- 3: Insulinpumpe
- 4: Datenverarbeitungseinrichtung
- 5: Kommunikations-Adapter
- 6: Datenübertragung
- 7: Übertragungskabel
- 8: Datenübertragung
- 9: Datenübertragung
- 10: Monitor

## Patentansprüche

1. Blutglukosesystem (1) zum Behandeln einer Glukosestoffwechselstörung, umfassend
- ein tragbares, eigenständig ambulant betreibbares Dosiergerät (3) zum Abgeben eines medizinischen Wirkstoffs für die Behandlung der Glukosestoffwechselstörung an einen Körper, das einen integrierten Zeitzähler zum Generieren von relativen Zeitwerten, eine Speichereinheit, in der Datensätze aus Dosiermengen und zugehörigen Zeitwerten abgespeichert werden und eine Vorrichtung zum Übertragen gespeicherter Datensätze an eine Datenverarbeitungseinrichtung (4) umfasst, und
- ein tragbares, eigenständig ambulant betreibbares Blutglukosemessgerät (2) zum Bestimmen des Blutglukosegehaltes des Körpers, das ein integriertes Zeitnormal und eine Speichereinheit, in der Datensätze aus Blutglukosemessungen und zugehörigen Zeitwerten abgespeichert werden, und eine Vorrichtung zum Übertragen gespeicherter Datensätze an eine Datenverarbeitungseinrichtung (4) umfasst,
- eine Datenverarbeitungseinrichtung (4) zum Verarbeiten von Datensätzen des Dosiergerätes (3) und des Blutglukosemessgerätes (2), mit einer Empfangsvorrichtung zum Empfangen der Datensätze von dem Dosiergerät (3) und dem Blutglukosemessgerät (2) sowie einer Recheneinheit zum Umwandeln der Zeitwerte der Datensätze des Dosiergerätes (3) in absolute Zeitwerte auf Basis eines Vergleiches des Zeitwertes des Zeitzählers des Dosiergerätes (3) mit dem Zeitwert eines Zeitnormals,
- wobei die Datenverarbeitungseinrichtung (4) dazu ausgebildet ist, die Datensätze des Dosiergerätes (3) und die Datensätze des Blutglukosemessgerätes (2) mittels des Zeitwertes des Zeitnormals durch eine zugeordnete Verknüpfung miteinander zu synchronisieren,
**dadurch gekennzeichnet, dass**
das Dosiergerät (3) einen Nadelsensor aufweist, mit dem erfassbar ist, ob sich während des Abgebens von Wirkstoff die verwendete Nadel im Körper befindet, und der Nadelsensor auch als Priming-Sensor verwendet wird, wobei der Bolus als Priming gewertet und der gespeicherte Datensatz entsprechend markiert wird, wenn ein Bolus abgegeben wird, ohne dass die Nadel in den Körper injiziert ist.

2. System (1) nach Anspruch 1, **dadurch gekennzeichnet, dass** als Zeitwerte für das Verknüpfen der Datensätze des Dosiergerätes (3) und des Blutglukosemessgerätes (2) die Zeitwerte des Zeitnormals des Blutglukosemessgerätes (3) verwendet werden.

3. System (1) nach einem der vorhergehenden Ansprüche, **dadurch gekennzeichnet, dass** die Datenverarbeitungseinrichtung (4) ein integriertes Zeitnormal umfasst und als Zeitwerte für das Verknüpfen der Datensätze des Dosiergerätes (3) und des Blutglukosemessgerätes (2) die Zeitwerte des Zeitnormals des Blutglukosemessgerätes (2) oder die Zeitwerte des Zeitnormals der Datenverarbeitungseinrichtung (4) verwendet werden oder eine Kombination von Zeitwerten beider Zeitnormale verwendet wird.

4. System (1) nach einem der vorhergehenden Ansprüche, **dadurch gekennzeichnet, dass** das Dosiergerät (3) ein Injektionsgerät, vorzugsweise ein Gerät zum Injizieren von Insulin ist.

5. System (1) nach einem der vorhergehenden Ansprüche, **dadurch gekennzeichnet, dass** das Übertragen der Datensätze von dem Blutglukosemessgerät (2) an die Datenverarbeitungseinrichtung (4) und/oder das Übertragen der Datensätze von dem Dosiergerät (3) an die Datenverarbeitungseinrichtung (4) mittels einer Kabelverbindung oder kabellos erfolgt.

6. Verfahren zum Verarbeiten von Analyseergebnissen mit einem Blutglukosesystem (1) zum Behandeln einer Glukosestoffwechselstörung, nach Anspruch 1, wobei das Blutglukosesystem (1)
- ein tragbares, eigenständig ambulant betreibbares Dosiergerät (3) zum Abgeben eines medizinischen Wirkstoffs für die Behandlung der Glukosestoffwechselstörung an einen Körper umfasst, das einen integrierten Zeitzähler zum Generieren von relativen Zeitwerten, eine Speichereinheit, in der Datensätze aus Dosiermengen und zugehörigen Zeitwerten abgespeichert werden und eine Vorrichtung zum Übertragen gespeicherter Datensätze an eine Datenverarbeitungseinrichtung (4) umfasst,
- ein tragbares, eigenständig ambulant betreibbares Blutglukosemessgerät (2) zum Bestimmen des Blutglukosegehaltes des Körpers umfasst, das ein integriertes Zeitnormal und eine Speichereinheit, in der Datensätze aus Blutglukosemessungen und zugehörigen Zeitwerten abgespeichert werden, und eine Vorrichtung zum Übertragen gespeicherter Datensätze an eine Datenverarbeitungseinrichtung (4) umfasst, und
- eine Datenverarbeitungseinrichtung (4) zum Verarbeiten von Datensätzen des Dosiergerätes (3) und des Blutglukosemessgerätes (2) umfasst, mit einer Empfangsvorrichtung zum Empfangen der Datensätze von dem Dosiergerät (3) und dem Blutglukosemessgerät (2) sowie einer Recheneinheit zum Umwandeln der Zeitwerte der Datensätze des Dosiergerätes (3) in absolute Zeitwerte auf Basis eines Vergleiches des Zeitwertes des Zeitzählers des Dosiergerätes (3) mit dem Zeitwert eines Zeitnormals,
- wobei die Datenverarbeitungseinrichtung (4) dazu ausgebildet ist, die Datensätze des Dosiergerätes (3) und die Datensätze des Blutglukosemessgerätes (2) mittels des Zeitwertes des Zeitnormals durch eine zugeordnete Verknüpfung miteinander zu synchronisieren,
umfassend die Verfahrensschritte
- Abspeichern eines oder mehrerer Datensätze in dem Dosiergerät (3) von mit dem Dosiergerät (3) durchgeführten Abgaben eines medizinischen Wirkstoffs für die Behandlung der Glukosestoffwechselstörung, beinhaltend Dosiermengen sowie Zeitwerte, die mittels des integrierten Zeitzählers zum Zeitpunkt der jeweiligen Abgabe gewonnen werden,
- Abspeichern eines oder mehrerer Datensätze in dem Blutglukosemessgerät (2) von mit dem Blutglukosemessgerät (2) durchgeführten Blutglukosemessungen, beinhaltend Analyseergebnisse sowie Zeitwerte, die mittels des integrierten Zeitnormals zum Zeitpunkt der jeweiligen Messung gewonnen werden,
- Übertragen eines oder mehrerer Datensätze des Blutglukosemessgerätes (2) und des Dosiergerätes (3) an die Datenverarbeitungseinrichtung (4) sowie Übergabe des aktuellen Zeitwertes des Zeitzählers des Dosiergerätes (3) an die Datenverarbeitungseinrichtung (4),
- Berechnen der absoluten Zeitwerte, zu denen der Wirkstoff von dem Dosiergerät (3) abgegeben wurde, mittels der Datenverarbeitungseinrichtung (4) aus den Zeitwerten des Datensatzes des Dosiergerätes (3) durch Vergleich des aktuellen Zeitwertes des Zeitzählers des Dosiergerätes (3) mit dem exakten Zeitwert eines Zeitnormals, wobei die Datensätze des Dosiergerätes (3) und die Datensätze des Blutglukosemessgerätes (2) mittels des Zeitwertes des Zeitnormals durch eine zugeordnete Verknüpfung miteinander synchronisiert werden, und
- Verarbeiten der Dosiermengen und Analyseergebnisse auf Basis der berechneten Zeitpunkte,
**dadurch gekennzeichnet, dass**
das Dosiergerät (3) einen Nadelsensor aufweist, der erfasst, ob sich während des Abgebens von Wirkstoff die verwendete Nadel im Körper befindet, und der Nadelsensor auch als Priming-Sensor verwendet wird, wobei der Bolus als Priming gewertet und der gespeicherte Datensatz entsprechend markiert wird, wenn ein Bolus abgegeben wird, ohne dass die Nadel in den Körper injiziert ist.

7. Verfahren nach Anspruch 6, **dadurch gekennzeichnet, dass** als Zeitwerte für das Verknüpfen der Datensätze des Dosiergerätes (3) und des Blutglukosemessgerätes (2) die Zeitwerte des Zeitnormals des Blutglukosemessgerätes (2) verwendet werden.

8. Verfahren nach Anspruch 6 oder 7, **dadurch gekennzeichnet, dass** ein Blutglukosemessgerät (1) verwendet wird, bei dem die Datenverarbeitungseinrichtung (4) ein integriertes Zeitnormal umfasst und zum Synchronisieren und Verknüpfen der Datensätze des Dosiergerätes (3) und des Blutglukosemessgerätes (2) unter Berechnung der Zeitpunkte, zu denen der Wirkstoff von dem Dosiergerät (3) abgegeben wurde, als Zeitnormal die Zeitwerte des Zeitnormals des Blutglukosemessgerätes (2) oder die Zeitwerte des Zeitnormals der Datenverarbeitungseinrichtung (4) verwendet werden oder eine Kombination von Zeitwerten beider Zeitnormale verwendet wird.

9. Verfahren nach einem der Ansprüche 6 bis 8, **dadurch gekennzeichnet, dass** aus dem Zeitwert des Zeitnormals zu einem bestimmten Zeitpunkt und dem Zeitwert des Zeitzählers zum gleichen Zeitpunkt eine Zeitdifferenz ermittelt wird und die Zeitwerte der Datensätze durch Addition der Zeitdifferenz in absolute Zeitwerte umgerechnet werden.

10. Verfahren nach Anspruch 9, **dadurch gekennzeichnet, dass** bei dem Übertragen gespeicherter Datensätze von dem Dosiergerät (3) an die Datenverarbeitungseinrichtung (4) die Zeitdifferenz zwischen dem Zeitzähler des Dosiergerätes (3) und dem Zeitnormal ermittelt wird und die ermittelte Zeitdifferenz gleich oder zu einem späteren Zeitpunkt zum Korrigieren der Zeitwerte der Datensätze des Dosiergerätes (3) verwendet wird.

11. Verfahren nach einem der Ansprüche 6 bis 10, **dadurch gekennzeichnet, dass** bei einer ersten Datenübertragung eine erste Zeitdifferenz zwischen Zeitnormal und Zeitzähler ermittelt wird und bei einer darauffolgenden zweiten Datenübertragung eine entsprechende zweite Zeitdifferenz und zwischen den Datenübertragungen liegende Zeitwerte durch Interpolation zwischen erster und zweiter Zeitdifferenz und Addition der interpolierten Zeitdifferenz zu dem Zeitwert der Dosisabgabe ermittelt werden.

12. Verfahren nach einem der Ansprüche 6 bis 11, **dadurch gekennzeichnet, dass** der Schritt des Verarbeitens von Dosiermengen und Analyseergebnissen das Abspeichern in einer Datenverarbeitungsvorrichtung, das Übertragen an eine Datenverarbeitungsvorrichtung oder das Darstellen der synchronisierten Datensätze des Dosiergerätes (3) mit einer Datenverarbeitungsvorrichtung und des Blutglukosemessgerätes (2) umfasst.

13. Verfahren nach einem der Ansprüche 6 bis 12, **dadurch gekennzeichnet, dass** der Zeitzähler mit einer vorbenannten Taktrate (v) zählt und die Berechnung der Dosierzeitpunkte (tA) aufgrund der Formel tA = tD - (nD - nA)/v erfolgt, wobei tD die am Zeitnormal zum Zeitpunkt der Datenübertragung vorliegende Zeit des Zeitnormals, nD die am Zeitzähler zum Zeitpunkt der Datenübertragung vorliegende Zahl, nA die zum Zeitpunkt der Dosisabgabe A gespeicherte Zahl des Zeitzählers und v die Taktrate des Zeitzählers ist.

14. Verfahren nach einem der Ansprüche 6 bis 13, **dadurch gekennzeichnet, dass** in dem von dem Dosiergerät (3) gespeicherten Datensatz die Dauer einer Dosierabgabe erfasst wird, vorzugsweise indem in dem Datensatz des Dosiergerätes (3) zu zugehörigen Zeitwerten jeweils der Beginn und das Ende einer Dosierabgabe vermerkt wird.

## Claims

1. A blood glucose system (1) for treating a glucose metabolic disorder, comprising
- a portable, autonomous, outpatient-operable dosing device (3) for delivering a medicinal active substance for treating glucose metabolic disorders in a body, which comprises an integrated time counter for generating relative time values, a storage unit in which data sets of dosing quantities and associated time values are stored and an apparatus for transferring stored data sets to a data processing device (4), and
- a portable, autonomous, outpatient-operable blood glucose meter (2) for determining the blood glucose content of the body, comprising an integrated time standard and a storage unit in which data sets from blood glucose measurements and associated time values are stored, and an apparatus for transferring stored data sets to a data processing device (4),
- a data processing device (4) for processing data sets of the dosing device (3) and the blood glucose meter (2), with a receiving apparatus for receiving the data sets from the dosing device (3) and the blood glucose measuring device (2) and a computing unit for converting the time values of the data sets of the dosing device (3) into absolute time values based on a comparison of the time value of the time counter of the dosing device (3) with the time value of a time standard,
- wherein the data processing device (4) is embodied to synchronise the data sets of the dosing device (3) and the data sets of the blood glucose meter (2) with each other by means of the time value of the time standard by an associated link,
**characterised in that**
the dosing device (3) has a needle sensor with which it is detectable whether the needle used is in the body during the delivery of an active substance, and the needle sensor is also used as a priming sensor, wherein the bolus is evaluated as priming and the stored data set is marked accordingly when a bolus is delivered without the needle being injected into the body.

2. The system (1) according to claim 1, **characterised in that** the time values of the time standard of the blood glucose meter (3) are used as time values for linking the data sets of the dosing device (3) and the blood glucose meter (2).

3. The system (1) according to any of the preceding claims, **characterised in that** the data processing device (4) comprises an integrated time standard and the time values of the time standard of the blood glucose meter (2) or the time values of the time standard of the data processing device (4) are used as time values for linking the data sets of the dosing device (3) and the blood glucose meter (2) or a combination of time values of both time standards is used.

4. The system (1) according to any of the preceding claims, **characterised in that** the dosing device (3) is an injection device, preferably a device for injecting insulin.

5. The system (1) according to any of the preceding claims, **characterised in that** the transferring of the data sets from the blood glucose meter (2) to the data processing device (4) and/or the transferring of the data sets from the dosing device (3) to the data processing device (4) occurs by means of a wire connection or wirelessly.

6. A method for processing analysis results with a blood glucose system (1) for treating a glucose metabolic disorder according to claim 1, wherein the blood glucose system (1)
- comprises a portable, autonomous, outpatient-operable dosing device (3) for delivering a medicinal active substance for treating the glucose metabolic disorder in a body, which comprises an integrated time counter for generating relative time values, a storage unit in which data sets of dosing quantities and associated time values are stored and an apparatus for transferring stored data sets to a data processing device (4),
- comprises a portable, autonomous, outpatient-operable blood glucose meter (2) for determining the blood glucose content of the body, comprising an integrated time standard and a storage unit in which data sets from blood glucose measurements and associated time values are stored, and an apparatus for transferring stored data sets to a data processing device (4), and
- comprises a data processing device (4) for processing data sets of the dosing device (3) and the blood glucose meter (2), with a receiving apparatus for receiving the data sets from the dosing device (3) and the blood glucose meter (2) and a computing unit for converting the time values of the data sets of the dosing device (3) into absolute time values based on a comparison of the time value of the time counter of the dosing device (3) with the time value of a time standard,
- wherein the data processing device (4) is embodied to synchronise the data sets of the dosing device (3) and the data sets of the blood glucose meter (2) with each other by means of the time value of the time standard by an associated link,
comprising the method steps of
- storing one or more data sets in the dosing device (3) of deliveries of a medicinal active substance for the treatment of the glucose metabolism disorder performed with the dosing device (3), containing dosing quantities and time values obtained by means of the integrated time counter at the time of the respective delivery,
- storing one or more data sets in the blood glucose meter (2) of blood glucose measurements performed with the blood glucose meter (2), containing analysis results and time values obtained by means of the integrated time standard at the time of the respective measurement,
- transferring one or more data sets of the blood glucose meter (2) and the dosing device (3) to the data processing device (4) and transfer of the current time value of the time counter of the dosing device (3) to the data processing device (4),
- calculating the absolute time values, at which the active substance was delivered by the dosing device (3), by means of the data processing device (4) from the time values of the data set of the dosing device (3) by comparison of the current time value of the time counter of the dosing device (3) with the exact time value of a time standard, wherein the data sets of the dosing device (3) and the data sets of the blood glucose meter (2) are synchronised with each other by means of the time value of the time standard by an associated link, and
- processing the dosing quantities and analysis results on the basis of the calculated times,
**characterised in that**
the dosing device (3) has a needle sensor which detects whether the needle used is in the body during the delivery of an active substance, and the needle sensor is also used as a priming sensor, wherein the bolus is evaluated as priming and the stored data set is marked accordingly when a bolus is delivered without the needle being injected into the body.

7. The method according to claim 6, **characterised in that** the time values of the time standard of the blood glucose meter (2) are used as time values for linking the data sets of the dosing device (3) and the blood glucose meter (2).

8. The method according to claim 6 or 7, **characterised in that** a blood glucose meter (1) is used, in which the data processing device (4) comprises an integrated time standard and the time values of the time standard of the blood glucose meter (2) or the time values of the time standard of the data processing device (4) are used as a time standard for synchronising and linking the data sets of the dosing device (3) and the blood glucose meter (2) upon calculation of the times at which the active substance was delivered by the dosing device (3), or a combination of time values of both time standards is used.

9. The method according to one of the claims 6 to 8, **characterised in that** a time difference is determined from the time value of the time standard at a certain time and the time value of the time counter at the same time and the time values of the data sets are converted into absolute time values by addition of the time difference.

10. The method according to claim 9, **characterised in that** the time difference between the time counter of the dosing device (3) and the time standard is determined during the transferring of stored data sets from the dosing device (3) to the data processing device (4) and the determined time difference is used, instantly or at a later time, for correcting the time values of the data sets of the dosing device (3).

11. The method according to one of the claims 6 to 10, **characterised in that** a first time difference between time standard and time counter is determined during a first data transfer and a corresponding second time difference and time values lying between the data transfers are determined during a subsequent second data transfer by interpolation between the first and second time difference and addition of the interpolated time difference to the time value of the dose delivery.

12. The method according to one of the claims 6 to 11, **characterised in that** the step of processing dosing quantities and analysis results comprises storing in a data processing device, the transferring to a data processing apparatus or displaying the synchronised data sets of the dosing device (3) with a data processing apparatus and the blood glucose meter (2).

13. The method according to one of the claims 6 to 12, **characterised in that** the time counter counts at an aforementioned clock speed (v) and the calculation of the dosing times (tA) occurs based on the formula tA = tD - (nD - nA)/v, wherein tD is the time of the time standard present at the time of data transfer, nD is the number present at the time counter at the time of data transfer, nA is the number of the time counter stored at the time of dose delivery A and v is the clock speed of the time counter.

14. The method according to one of the claims 6 to 13, **characterised in that** the duration of a dosing delivery is recorded in the data set stored by the dosing device (3), preferably by recording the start and end of a dosing delivery in each case in the data set of the dosing device (3) for associated time values.

## Revendications

1. Système de gestion du taux de glycémie (1), destiné au traitement d'un trouble du métabolisme du glucose, comprenant
- un dispositif doseur (3) portable, à fonctionnement autonome ambulant, destiné à délivrer à un corps un principe actif médical pour le traitement du trouble du métabolisme du glucose, qui comprend un compteur de temps intégré, destiné à générer des valeurs temporelles relatives, une unité mémoire, dans laquelle sont mémorisés des ensembles de données issus de quantités dosées et de valeurs temporelles correspondantes et un dispositif pour la transmission d'ensembles de données mémorisés à un système de traitement de données (4) et
- un dispositif de mesure du taux de glycémie (2) portable, à fonctionnement autonome ambulant, destiné à déterminer la teneur en glucose sanguin dans le corps, qui comprend un étalon de temps intégré et une unité mémoire, dans laquelle sont mémorisés des ensembles de données issus de mesures du taux de glycémie et des valeurs temporelles correspondantes et un dispositif pour transférer des ensembles de données mémorisés à un système de traitement de données (4),
- un système de traitement de données (4), destiné à traiter des ensembles de données du dispositif doseur (3) et du dispositif de mesure du taux de glycémie (2), doté d'un dispositif récepteur, destiné à recevoir les ensembles de données du dispositif doseur (3) et du dispositif de mesure du taux de glycémie (2), ainsi qu'une unité de calcul, destinée à convertir les valeurs temporelles des ensembles de données du dispositif doseur (3) en des valeurs temporelles absolues, sur la base d'une comparaison de la valeur temporelle du compteur de temps du dispositif doseur (3) avec la valeur temporelle d'un étalon de temps,
- le système de traitement de données (4) étant conçu pour synchroniser les uns avec les autres les ensembles de données du dispositif doseur (3) et les ensembles de données du dispositif de mesure du taux de glycémie (2) au moyen de la valeur temporelle de l'étalon de temps par une interconnexion associée,
**caractérisé en ce que**
le dispositif doseur (3) comporte un capteur d'aiguille permettant de détecter si, pendant la délivrance de principe actif, l'aiguille utilisée se trouve dans le corps et **en ce que** le capteur d'aiguille est également utilisé en tant que capteur d'amorce, le bolus étant évalué en tant qu'amorce et l'ensemble de données mémorisé étant marqué en conséquence si un bolus est délivré sans que l'aiguille soit insérée dans le corps.

2. Système (1) selon la revendication 1, **caractérisé en ce qu'**en tant que valeurs temporelles pour l'interconnexion des ensembles de données du dispositif doseur (3) et du dispositif de mesure du taux de glycémie (2), les valeurs temporelles de l'étalon de temps du dispositif de mesure du taux de glycémie (3) sont utilisées.

3. Système (1) selon l'une quelconque des revendications précédentes, **caractérisé en ce que** le système de traitement de données (4) comprend un étalon de temps intégré et en tant que valeurs temporelles pour l'interconnexion des ensembles de données du dispositif doseur (3) et du dispositif de mesure du taux de glycémie (2), les valeurs temporelles de l'étalon de temps du dispositif de mesure du taux de glycémie (2) ou les valeurs temporelles de l'étalon de temps du système de traitement de données (4) sont utilisées ou une association de valeurs temporelles des deux étalons de temps est utilisée.

4. Système (1) selon l'une quelconque des revendications précédentes, **caractérisé en ce que** le dispositif doseur (3) est un dispositif d'injection, de préférence, un dispositif destiné à injecter de l'insuline.

5. Système (1) selon l'une quelconque des revendications précédentes, **caractérisé en ce que** le transfert des ensembles de données du dispositif de mesure du taux de glycémie (2) au système de traitement de données (4) et/ou le transfert des ensembles de données du dispositif doseur (3) au système de traitement de données (4) s'effectue par connexion filaire ou sans fil.

6. Procédé destiné à traiter des résultats d'analyses avec un système de gestion du taux de glycémie (1) pour le traitement d'un trouble du métabolisme du glucose selon la revendication 1, le système de gestion du taux de glycémie (1), comprenant
- un dispositif doseur (3) portable, à fonctionnement autonome ambulant, destiné à délivrer à un corps un principe actif médical pour le traitement du trouble du métabolisme du glucose, qui comprend un compteur de temps intégré, destiné à générer des valeurs temporelles relatives, une unité mémoire, dans laquelle sont mémorisés des ensembles de données issus de quantités dosées et de valeurs temporelles correspondantes et un dispositif pour la transmission d'ensembles de données mémorisés à un système de traitement de données (4),
- un dispositif de mesure du taux de glycémie (2) portable, à fonctionnement autonome ambulant, destiné à déterminer la teneur en glucose sanguin dans le corps, qui comprend un étalon de temps intégré et une unité mémoire, dans laquelle sont mémorisés des ensembles de données issus de mesures du glucose sanguin et des valeurs temporelles correspondantes et un dispositif pour transférer des ensembles de données mémorisés à un système de traitement de données (4), et
- un système de traitement de données (4), destiné à traiter des ensembles de données du dispositif doseur (3) et du dispositif de mesure du taux de glycémie (2), doté d'un dispositif récepteur, destiné à recevoir les ensembles de données du dispositif doseur (3) et du dispositif de mesure du taux de glycémie (2), ainsi qu'une unité de calcul, destinée à convertir les valeurs temporelles des ensembles de données du dispositif doseur (3) en des valeurs temporelles absolues, sur la base d'une comparaison de la valeur temporelle du compteur de temps du dispositif doseur (3) avec la valeur temporelle d'un étalon de temps,
- le système de traitement de données (4) étant conçu pour synchroniser les uns avec les autres les ensembles de données du dispositif doseur (3) et les ensembles de données du dispositif de mesure du taux de glycémie (2) au moyen de la valeur temporelle de l'étalon de temps par une interconnexion associée, comportant les étapes consistant à
- mémoriser un ou plusieurs ensembles de données dans le dispositif doseur (3) de délivrances réalisées à l'aide du dispositif doseur (3) d'un principe actif médical destiné au traitement du trouble du métabolisme du glucose, comprenant des quantités dosées, ainsi que des valeurs temporelles, qui au moyen du compteur de temps intégré, sont acquises au moment de la délivrance respective,
- mémoriser un ou plusieurs ensembles de données dans le dispositif de mesure du taux de glycémie (2) des mesures du taux de glycémie réalisées avec le dispositif de mesure du taux de glycémie (2), comprenant des résultats d'analyses, ainsi que des valeurs temporelles, qui sont acquises au moyen de l'étalon de temps intégré au moment de la mesure respective,
- transférer un ou plusieurs ensembles de données du dispositif de mesure du taux de glycémie (2) et du dispositif doseur (3) au système de traitement de données (4), ainsi que transférer la valeur temporelle actuelle du compteur de temps du dispositif doseur (3) au système de traitement de données (4),
- calculer les valeurs temporelles absolues, auxquelles le principe actif a été délivré par le dispositif doseur (3), au moyen du système de traitement de données (4) à partir des valeurs temporelles de l'ensemble de données du dispositif doseur (3) par comparaison de la valeur temporelle actuelle du compteur de temps du dispositif doseur (3) avec la valeur temporelle exacte d'un étalon de temps, sachant que les ensembles de données du dispositif doseur (3) et les ensembles de données du dispositif de mesure du taux de glycémie (2) sont synchronisés les uns avec les autres au moyen de la valeur temporelle de l'étalon de temps par une interconnexion associée, et
- traiter les quantités dosées et les résultats d'analyses sur la base des moments calculés,
**caractérisé en ce que**
le dispositif doseur (3) comporte un capteur d'aiguille qui détecte si, pendant la délivrance du principe actif, l'aiguille utilisée se trouve dans le corps et **en ce que** le capteur d'aiguille est également utilisé en tant que capteur d'amorce, le bolus étant évalué en tant qu'amorce et l'ensemble de données mémorisé étant marqué en conséquence si un bolus est délivré, sans que l'aiguille ne soit insérée dans le corps.

7. Procédé selon la revendication 6, **caractérisé en ce qu'**en tant que valeurs temporelles pour l'interconnexion des ensembles de données du dispositif doseur (3) et du dispositif de mesure du taux de glycémie (2), on utilise les valeurs temporelles de l'étalon de temps du dispositif de mesure du taux de glycémie (2).

8. Procédé selon la revendication 6 ou 7, **caractérisé en ce qu'**on utilise un dispositif de mesure du taux de glycémie (1) sur lequel le système de traitement de données (4) comprend un étalon de temps intégré et pour la synchronisation et l'interconnexion des ensembles de données du dispositif doseur (3) et du dispositif de mesure du taux de glycémie (2) en calculant les moments auxquels le principe actif a été délivré par le dispositif doseur (3) en tant qu'étalon de temps, les valeurs temporelles de l'étalon de temps du dispositif de mesure du taux de glycémie (2) ou les valeurs temporelles de l'étalon de temps du système de traitement de données (4) sont utilisées ou une association de valeurs temporelles des deux étalons de temps est utilisée.

9. Procédé selon l'une quelconque des revendications 6 à 8, **caractérisé en ce qu'**à partir de la valeur temporelle de l'étalon de temps à un certain moment et la valeur temporelle du compteur de temps au même moment, une différence de temps est déterminée et les valeurs temporelles des ensembles de données sont converties par addition de la différence de temps en valeurs temporelles absolues.

10. Procédé selon la revendication 9, **caractérisé en ce que** lors du transfert d'ensembles de données mémorisés du dispositif doseur (3) au système de traitement de données (4), la différence de temps entre le compteur de temps du dispositif doseur (3) et l'étalon de temps est déterminée et la différence de temps déterminée est utilisée tout de suite ou plus tard pour corriger les valeurs temporelles des ensembles de données du dispositif doseur (3).

11. Procédé selon l'une quelconque des revendications 6 à 10, **caractérisé en ce que** lors d'un premier transfert de données, une première différence de temps entre un étalon de temps et le compteur de temps est déterminée et lors d'un deuxième transfert de données consécutif, une deuxième différence de temps correspondante et des valeurs temporelles situées entre les transferts de données sont déterminées par interpolation entre la première et la deuxième différence de temps et addition de la différence de temps interpolée à la valeur temporelle de la délivrance de la dose.

12. Procédé selon l'une quelconque des revendications 6 à 11, **caractérisé en ce que** l'étape du traitement de quantités dosées et de résultats d'analyses comprend la mémorisation dans un dispositif de traitement de données, le transfert à un dispositif de traitement de données ou la représentation des ensembles de données synchronisés du dispositif doseur (3) à l'aide d'un dispositif de traitement de données et du dispositif de mesure du taux de glycémie (2).

13. Procédé selon l'une quelconque des revendications 6 à 12, **caractérisé en ce que** le compteur de temps compte à un une fréquence d'horloge précitée (v) et le calcul des moments d'administration (tA) s'effectue sur la base de la formule tA = tD - (nD - nA)/v, sachant que tD est le temps de l'étalon de temps présent sur l'étalon de temps au moment du transfert de données, nD est le nombre présent sur le compteur de temps au moment du transfert de données, nA est le chiffre mémorisé du compteur de temps au moment de la délivrance de la dose A et v est la fréquence d'horloge du compteur de temps.

14. Procédé selon l'une quelconque des revendications 6 à 13, **caractérisé en ce que** dans l'ensemble de données mémorisé par le dispositif doseur (3) la durée d'une délivrance de dose est détectée, de préférence, **en ce que** dans l'ensemble de données du dispositif doseur (3), respectivement le début et la fin d'une délivrance de dose est annoté(e) aux valeurs temporelles correspondantes.
